# EUROPEAN PATENT APPLICATION

(11) **EP 3 557 572 A1**
(43) Date of publication of application: **23.10.2019**
(21) Application number: 19168734.2
(22) Date of filing: 11.04.2019
(51) Int. Cl.: G10K 11/178, G10K 11/34, H04R 1/02, H04R 3/00, H04R 3/12, H04R 1/40, A61F 5/56, A47C 21/00, H04S 7/00

(54) **NOISE REDUCTION SYSTEM**

(30) Priority: 19.04.2018 DK PA201800175
(71) Applicant: Nomoresnore Ltd., London N3 2LJ (GB)
(72) Inventor: Allen, Raymond, London Colney, Hertfordshire AL2 1GA (GB)
(74) Representative: Andreasen, Søren Laursen Vasegaard

(57) **Abstract**

A noise reduction system (2) for reducing at least one sound signal (12), wherein the noise reduction system (2) is disclosed. The noise reduction system (2) comprises at least one directional microphone (8, 8', 8", 8"') for detecting at least one sound signal (12) to be reduced, a processing unit (38) for generating at least one cancellation signal (14) and at least one directional speaker unit (10, 10', 10", 10"') for emitting the at least one cancellation signal (14). The noise reduction system (2) comprises a head detection sensor (22) configured to detect the position of the head (32) of a user (4, 4').

## Description

### Field of invention

The present invention relates to a noise reduction system for reducing at least one sound signal. The present invention more particularly, relates to a noise reduction system for reducing the sound of snoring.

### Prior art

Snoring can be extremely loud and a huge challenge for a partner lying next to a snorer. Snoring causes sleep deprivation to the snorers and to the person who has to listen to their snoring. Several studies have revealed a correlation between loud snoring and the risk of heart attack as well as stroke. Snoring can cause daytime drowsiness and a reduced ability to focus due to the poor sleep.

There is no known treatment that can completely stop snoring. Some of the known treatments for snoring include devices that the snorer has to wear or that has to be arranged on the snorer. These devices, however, typically are uncomfortable to wear. It is also to be mentioned, that a bed companion may develop fear in the time between snores, and may wake up and fear that that the sudden silence is a symptom of an ailment.

It is also known that sleep apnoea may disturb a bed companion as the apnoea may cause a bed companion to wake up with anxiety over the well being of a companion suffering from the condition and thus has a range of prolonged breathing pauses during sleep. If sleep apnoea is combined with snoring, which is not unheard of, the situation worsens for the bed companion.

Thus, there is a need for a noise reduction system and a method which reduces or even eliminates the above-mentioned disadvantages of the prior art.

### Summary of the invention

The object of the present invention can be achieved by a noise reduction system as defined in claim 1 and by a method as defined in claim 8. Preferred embodiments are defined in the dependent subclaims, explained in the following description and illustrated in the accompanying drawings.

The noise reduction system according to the invention is a noise reduction system for reducing at least one sound signal, wherein the noise reduction system comprises:
- several directional microphones spaced from each other and adapted to detect at least one location specific sound signal to be reduced;
- a signal processing unit adapted to generate at least one cancellation signal;
- a plurality of directional speaker units adapted to emit at least one cancellation signal;
- a head detection sensor configured to detect the position of the head of a user;
- whereby further the signal processing unit is configured to determine which one or more of the location specific sound signals picked up by the directional microphones are to be used in order to generate and emit the cancellation signal for providing a noise reduction and;
- wherein the directional microphones are arranged to pick up sound from different directions and;
- wherein further the signal processing unit is also adapted to determine which of the directional speaker units should be used for emitting the cancellation signal and;
- wherein the directional speaker units are arranged to emit sound towards different directions.

Hereby, it is possible to provide a noise reduction system that reduces the snoring sound in a non-invasive manner that does not require any devices causing inconvenience for the snorer. Additionally, the noise reduction system is capable of reducing other undesired sounds such as sounds from traffic, neighbours or music being played in an adjacent room. The selection among the incoming microphone signal and among the possible speaker units is made such that the most effective cancelation sound is obtained and thus an optimal noise reduction is obtained. This optimization may preferably be based on a desired property of the sound reaching the ear of a bed companion, such as minimizing energy of this sound at all times or minimizing energy at certain frequency ranges of this sound.

The processing unit may include a processor of any suitable type and size.

The at least one directional speaker unit may be any type of directional speaker unit suitable for emitting the at least one cancellation signal.

The head detection sensor may be any type of sensor being capable of detecting the position of the head of a user. By the term "the position of the head" is meant the direction relative to the sensor and the distance from the sensor.

It may be advantageous that the head detection sensor is an optical sensor configured to detect the position of the head. The sensor may apply techniques disclosed in one or more of the following granted US patents: US8989455B2, US9589177B2 and US9818023B2. By using such technique, it is possible to provide a head position detection in a simple manner.

It may be an advantage that the head detection sensor is an optical sensor configured to detect the position of the head in the night time. In a preferred embodiment, the sensor comprises an infrared detection member. The infrared detection member may be an infrared reflective optical detector.

When the noise reduction system comprises several directional microphones spaced from each other, it is possible to provide a more accurate detection of the sound present at the ear region of the person that is exposed to snoring. The directional microphones are arranged to pick up sound from different directions.

When the noise reduction system comprises several directional speaker-units spaced from each other, it is possible to emit a more effective cancellation signal.

It may be an advantage that the directional speaker units and/or the directional microphones are moveably arranged. Hereby, it is possible to adjust the position and/or orientation of the directional speaker units and/or the directional microphones in order to pick up a sound signal at an ear of the person and/or to emit the cancellation signal towards an ear of the person in a more accurate manner.

In one embodiment, the position and/or orientation of the directional speaker units and/or the directional microphones are configured to be manually changed by means of one or more adjustment members.

It may be advantageous that the noise reduction system comprises one or more actuators arranged to change the position or orientation of one or more of the directional speaker units and/or one or more of the directional microphones. Hereby, the position or orientation of the directional speaker units and/or the directional microphones can be automatically adjusted. Accordingly, it is possible to pick up a sound signal at an ear of the person and/or to emit the cancellation signal towards an ear of the person in a more accurate manner.

It is preferred that the one or more actuators (e.g. electrical actuators) are controlled by the processing unit on the basis of the detected signals of the sensor(s) (the detected position of the head).

In one embodiment, the noise reduction system comprises a housing formed as an elongated structure.

It may be beneficial that the noise reduction system comprises one or more additional hi-fi speakers. Hereby, it is possible to use the noise reduction system to play music or other sounds when the noise reduction system is not being used to reduce an undesired sound.

In one embodiment, the noise reduction system comprises an antenna and structures allowing the noise reduction system to be configured remotely e.g. by using a smartphone.

In one embodiment, the noise reduction system comprises a sound unit configured to generate an acoustic sound signal to be emitted by at least one directional speaker unit. Hereby, it is possible to generate an alarm to be heard by a single person (e.g. laying in a bed) only. Accordingly, it is possible to wake up a first person sleeping in a bed without waking up a second person lying next to the first person. The acoustic sound signal may be any kind of signal such as an alarm clock sound, a human voice or music. The alarm signal may be supplemented with or exchanged with a light signal. This could be a LED narrow beam light signal added at the location of the head of a user.

In one embodiment, the noise reduction system comprises a sound unit configured to generate an acoustic sound signal to be emitted by one or more hi-fi speakers. This may be an advantage if the noise reduction system is intended for generating an alarm to wake up two persons in a single bed at the same time.

The sound unit may be configured to generate the acoustic sound signal on a predefined time. A predefined time may be the time to wake up in the morning by way of example. It may be advantageous that the noise reduction system is configurable so that the time for generating the alarm can be set by the user of the noise reduction system.

In an embodiment, the noise reduction system in addition to or alternatively comprises an electric lamp, such as a LED and directional lamp, which shall turn on along with or as an alternative to waking up calls from an acoustic alarm.
The method according to the invention is a method for reducing at least one location specific sound signal picked up by a plurality of directional microphones, said method comprises the step of:
- generating, in a signal processing unit, at least one cancellation signal;
- emitting at least on cancellation signal by means of a plurality of directional speaker units,
wherein the method comprises the further step of detecting the position of the head of a user and determining towards which direction the cancellation signal has to be emitted, and whereby further:
- the signal processing unit determines which one or more of the location specific sound signals picked up by the directional microphones are to be used in order to generate and emit the cancellation signal for providing the noise reduction and;
- wherein it is determined which ones of the plurality of directional speaker units are to be used in order to emit the cancellation signal and;
- wherein the directional microphones pick up sound from different directions and the directional speaker units emit sound towards different directions.

Hereby, the method provides an improved way of reducing a detected sound, wherein the methods takes the position of the head into account and emits the cancellation signal in a direction towards the head. The plurality of directional speaker units is utilized to enable a better dampening signal being transmitted towards the targeted area.

Preferably, on the basis of the detected position of a head, one or more sound signals picked up by a plurality of different directional microphones are selected and a cancellation signal is generated to reduce the selected one or more sound signals. Hereby, the method enables a more accurate determination of the noise signal that has to be reduced. Accordingly, a more accurate (and efficient) cancellation signal can be generated and emitted by using the method. As mentioned above, efficiency is determined according to a chosen scheme, such as minimization of overall energy or minimization of energy levels of chosen frequency ranges of a resulting sound, which reaches the ear or ears of person.

### Description of the Drawings

The invention will become more fully understood from the detailed description given herein below. The accompanying drawings are given by way of illustration only, and thus, they are not limitative of the present invention. In the accompanying drawings:
- Fig. 1A: shows a schematic side view of a noise reduction system according to the invention;
- Fig. 1B: shows a close-up view of a cancellation signal emitted towards the ear of a person;
- Fig. 2A: shows a graph depicting the amplitude of a noise signal versus time, wherein the noise signal is picked up by means of one or more directional microphones of a noise reduction system according to the invention;
- Fig. 2B: shows a graph depicting a cancellation signal emitted by one or more directional speaker units of a noise reduction system according to the invention;
- Fig. 2C: shows a graph depicting a noise reduced signal corresponding to the sound signal that is received by the user of the noise reduction system according to the invention;
- Fig. 3A: shows a perspective view of a noise reduction system according to the invention mounted on a headboard of a bed;
- Fig. 3B: shows a front view of the noise reduction system that basically corresponds to the one shown in Fig. 3A;
- Fig. 4A: shows a noise reduction system according to the invention;
- Fig. 4B: shows another noise reduction system according to the invention;
- Fig. 4C: shows a further noise reduction system according to the invention;
- Fig. 4D: shows an even further noise reduction system according to the invention;
- Fig. 4E: shows another noise reduction system according to the invention;
- Fig. 4F: shows a noise reduction system according to the invention, wherein the noise reduction system comprises two sets of additional high fidelity (hi-fi) speakers;
- Fig. 5A: shows a top view of a bed having a headboard, wherein a noise reduction system according to the invention is mounted on the headboard;
- Fig. 5B: shows a noise reduction system according to the invention comprising an arced housing;
- Fig. 6: shows a schematic view of a noise reduction system according to the invention and
- Fig. 6B: shows a view of a cross-sectional view of a noise reduction system according to the invention.

### Detailed description of the invention

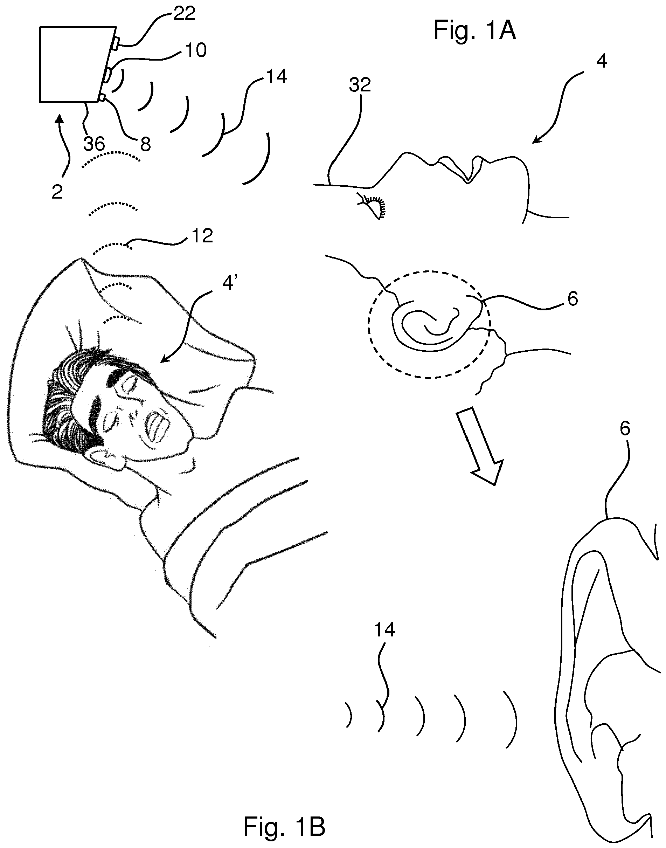

Referring now in detail to the drawings for the purpose of illustrating preferred embodiments of the present invention, a noise reduction system 2 of the present invention is illustrated in Fig. 1A.

Fig. 1A is a schematic side view of a noise reduction system 2 according to the invention. The noise reduction system 2 comprises a housing 36 that can be arranged in any suitable position such as on the headboard of a bed or a wall close to the head end of a bed. The housing 36 constitutes an elongated structure (that may be referred to as a sound bar).

The noise reduction system 2 further comprises a plurality of directional microphones 8 arranged in various positions of the housing 36. Accordingly, the directional microphones 8 are configured to pick up sound signals from different predefined areas (at different angular positions). Therefore, the noise reduction system 2 picks up a plurality of location specific sound signals 12.

The noise reduction system 2 furthermore comprises a plurality of directional speaker units 10 arranged in different positions of the housing 36. The directional speaker units 10 are configured to emit sound signals towards different predefined areas (at different angular positions).

The noise reduction system 2 also comprises a sensor 22 for detecting the position of the head 32 of a person 4 (e.g. laying in a bed in which the noise reduction system 2 is used).

The noise reduction system 2 is configured to reduce an undesired noise such as snoring. In order to reduce the snoring, the noise reduction system 2 detects the position of the head 32 of a first person 4 that is troubled by the snoring noise 12 generated by a second person 4' lying next to the first person 4. The position of the head 32 is detected by means of the sensor 22 that may be an optical sensor configured to detect the position of the head 32 by using the principles described in one or more of the following granted US patents US8989455B2, US9589177B2 and US9818023B2.

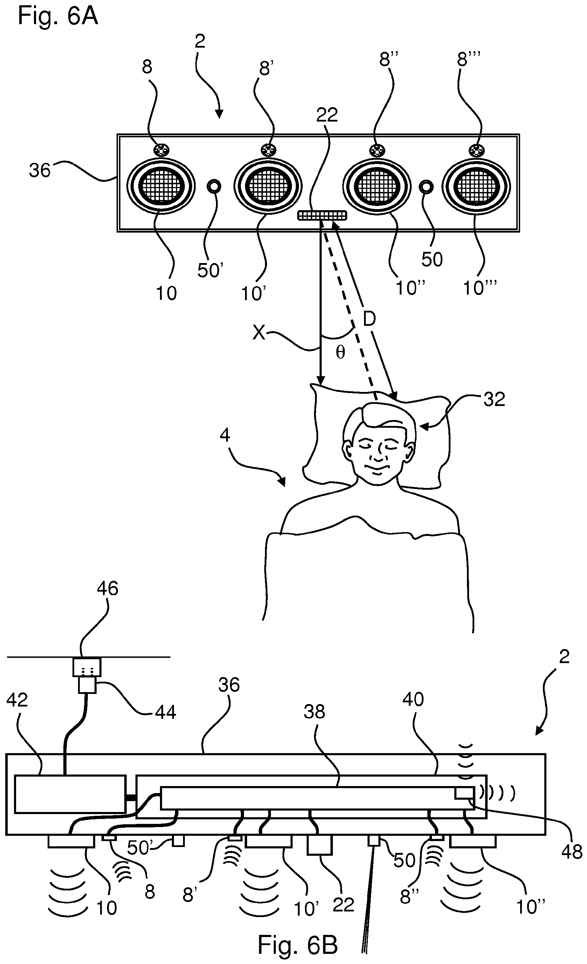

When the position of the head 32 has been detected, a processing unit (see Fig. 6B) of the noise reduction system 2 determines which of the location specific sound signals 12 to be used in order to generate and emit the most effective cancellation signal 14 for providing the most optimum noise reduction. It is possible to use a single location specific sound signal 12 or to mix (e.g. components) several location specific sound signals 12. The selection of the one or more specific sound signals 12 to be used may preferably be carried out by the processing unit by using a predefined algorithm. The predefined algorithm may comprise a digitizing step and a digital filtering step. Analog filtering without the digitizing step is also a possible option, and in systems for active noise damping, it is often preferred as processing time may be short compared to processing time in digital filtering, and short processing times are essential in order to generate an efficient noise cancellation signal. Also combinations of digital and analog filtering systems are known.

The processing unit of the noise reduction system 2 also determines which of the directional speaker units 10 should be used for emitting the most effective cancellation signal 14. A single directional speaker unit 10 or several directional speaker units 10 may be applied for this purpose.

The directional speaker units may be of the type based on photoacoustics. In this technique a laser beam is either sweeped or power modulated, and by choosing a laser wavelength which agitates or energize water molecules in the air, the sweeping or modulation done at sound frequency may produce sound in the audible range. The method was newly developed at Massechusetts Institute of Technology's Lincoln Laboratory and allows for highly directional sound production, using one or more directional laser beams. An advantage with the developed technique is that the produced sound may only be heard at a certain distance from the transmitter of the laser beam, which makes this particular sound production method especially suited in an effort to produce a highly localized cancellation signal, such as a cancellation signal targeted at one or both ears of a bed companion suffering from loud snoring noises from the adjacent sleeper. At present, it is reported, that the range from transmitter to where the sound will be generated is limited, however in this particular case, this is not an issue, as the directional speakers shall reside only a short length from the ear at which the cancellation signal is to be active.

Parametric speaker units are also usable in this connection and included as possible directional speaker units.

In one embodiment, a single directional speaker unit 10 is used to emit a cancellation signal 14. In another embodiment, several directional speaker units 10 are used to emit a cancellation signal 14. The cancellation signal 14 is emitted towards the head 32 and thus it is picked up by an ear 6 of the person 4.

The noise reduction system 2 is configured to keep this process going on a continuous basis. Accordingly, if the first person moves the head 32, the noise reduction system 2 will detect the new position of the head 32. Based on the new position of the head 32, the noise reduction system 2 will determine the one or more location specific sound signals 12 to be used for generating and emitting the signal 14 so that the noise reduction can be provided.

The most effective cancellation sound 14 is desired in order to obtain the most optimum noise reduction. This may be obtained by minimizing the overall energy in the sound remaining after the noise reduction sound and noise sound (snoring sound) have been mixed at the ear of a sleeping bed companion.
Fig. 1B illustrates a close-up view of a cancellation signal 14 emitted towards the ear 6 of the person 4 shown in Fig. 1A. It can be seen that the cancellation signal 14 can reach the inner ear through the ear canal.

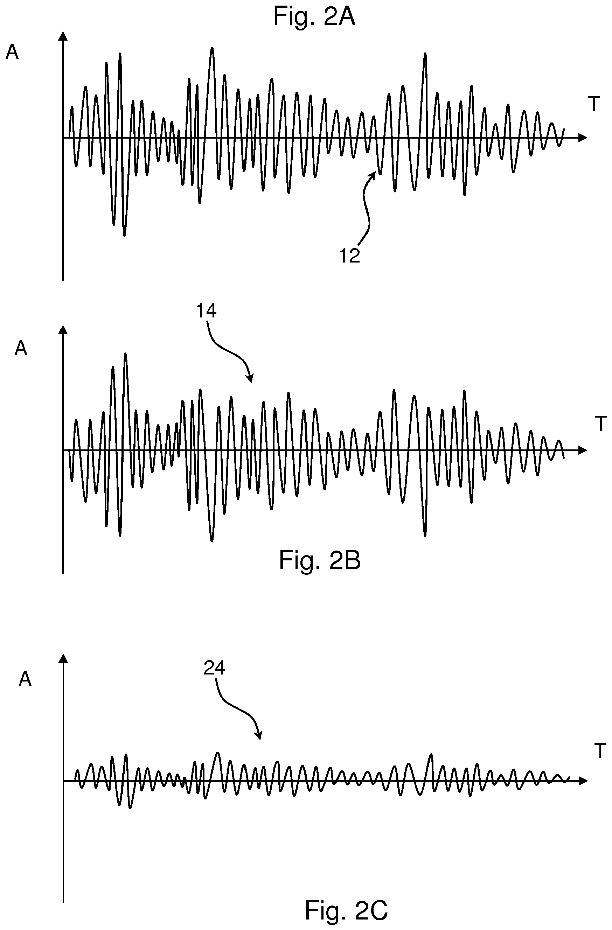

Fig. 2A illustrates a graph depicting the amplitude A of a noise signal 12 versus time T, wherein the noise signal 12 is picked up by means of r more directional microphones of a noise reduction system according to the invention. The noise signal 12 may represent snoring like illustrated in Fig. 1A. Accordingly, the noise signal 12 may be picked up by a single directional microphone. It is, however, also possible to mix sound signals picked up by several directional microphones and that the noise signal 12 represents a mix of snoring sound signals picked up by using various directional microphones as explained with reference to Fig. 1A.

Fig. 2B illustrates a graph depicting a cancellation signal 14 emitted by one or more directional speaker unit of a noise reduction system according to the invention. The cancellation signal 14 may be configured to cancel out snoring like explained with reference to Fig. 1A.

The cancellation signal 14 may be emitted by a single directional speaker unit. Alternatively, the cancellation signal 14 may be a mix of several cancellation signals 14 emitted by several directional speaker units.

Fig. 2C illustrates a graph depicting a noise reduced signal 24 corresponding to the sound signal that is received by the user of the noise reduction system according to the invention. When comparing the amplitude of the graphs illustrated in Fig. 2A, Fig. 2B and Fig. 2C, it can be seen that the noise reduced signal 24 has a smaller amplitude than the noise signal 12 (shown in Fig. 2A). Accordingly, the noise signal 12 is significantly reduced by using the noise reduction system according to the invention.

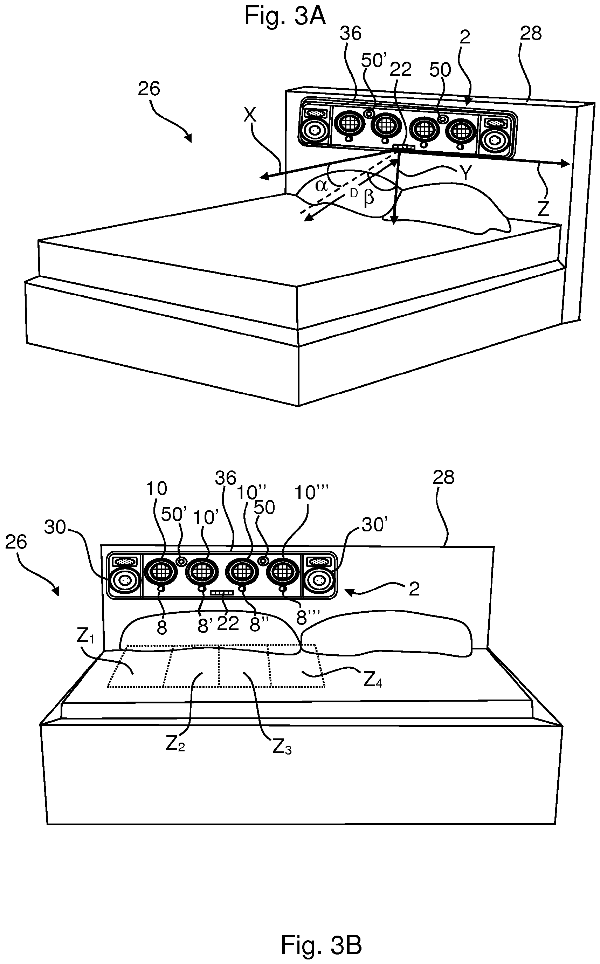

Fig. 3A illustrates a perspective view of a noise reduction system 2 according to the invention mounted on a headboard 28 of a bed 26. Alternatively, the noise reduction system 2 may be arranged on a wall in case that the bed 26 has no headboard.

The noise reduction system 2 comprises a housing 36 formed as an elongated structure. The housing 36 is basically box-shaped, however, its thickness is small compared to its length (its extent along the horizontal Z-axis) and its height (its extent along the vertical Y-axis).

The noise reduction system 2 comprises a sensor 22 arranged and configured to detect the head of a person (not shown) laying in the bed 26. The position of the head may be expressed in terms of the angular position of the head measured with respect to the Cartesian coordinate system comprising the axis X, Y, Z. The position of the head can be uniquely determined when the following three quantities are known:
- the distance D between the head and the sensor 22 and
- the angle α between the X axis and the direction (indicated by a dotted line) in which the head is positioned relative to the sensor 22 and
- the angle β between the Y axis and the direction in which the head is positioned relative to the sensor 22.

The sensor 22 may be an optical sensor configured to determine the position of the head by using the principles described in one or more of the following granted US patents US8989455B2, US9589177B2 and US9818023B2.

Fig. 3B illustrates a front view of the noise reduction system 2 that basically corresponds to the one shown in Fig. 3A. It can be seen that the noise reduction system 2 is attached to the headboard 28 of a bed 26. The noise reduction system 2 comprises an elongated housing 36 equipped with a centrally arranged sensor 22 configured to detect the head of a person positioned close (preferably within the range of 40-120 cm) to the noise reduction system 2.

The noise reduction system 2 additionally comprises four directional microphones 8, 8', 8", 8"' evenly distributed along the length of the housing 36. These directional microphones 8, 8', 8", 8"' are arranged to detect sound from various zones Z₁, Z₂, Z₃, Z₄. The first directional microphone 8 is arranged and configured to detect sound from a first zone Z₁. The second directional microphone 8' is arranged and configured to detect sound from a second zone Z₂. The third directional microphone 8" is arranged and configured to detect sound from a third zone Z₃, whereas the fourth directional microphone 8"' is arranged and configured to detect sound from a fourth zone Z₄.

The noise reduction system 2 comprises four directional speaker units 10, 10', 10", 10"' evenly distributed along the length of the housing 36. The directional speaker units 10, 10', 10", 10'" are arranged to emit sound towards the zones Z₁, Z₂, Z₃, Z₄. The first directional speaker unit 10 is arranged and configured to emit sound towards the first zone Z₁. The second directional speaker unit 10' is arranged and configured to emit sound towards the second zone Z₂. The third directional speaker unit 10" is arranged and configured to emit sound towards the third zone Z₃, whereas the fourth directional speaker unit 10"' is arranged and configured to emit sound towards the fourth zone Z₄.

The noise reduction system 2 comprises a first additional hi-fi speaker 30 and a second additional hi-fi speaker 30'. The hi-fi speakers 30, 30' are arranged in opposing end portions of the housing 36 of the noise reduction system 2. The noise reduction system 2 is configured to emit sound signals such as music by means of the hi-fi speakers 30, 30'.

The noise reduction system 2 comprises a sound unit (not shown) configured to generate an alarm to be emitted by one or more of the directional speaker units 10, 10', 10", 10"'. Accordingly, it is possible to generate an alarm that can only be heard by one out of two persons laying in the bed 26. Accordingly, it is possible to wake up a first person sleeping in the bed 26 without waking up a second person lying next to the first person. Additionally a narrow beam LED light may be added to the noise reduction system 2 in order to enhance the alarm directed at a first person in the bed without waking up a second person lying next to the first person. This allows the person in question to choose the nature of the alarm or alarm combination which is the most comfortable or most efficient. The LED light source 50, 50' is disclosed in Fig. 3A, 3B, 4D, 4F, 5B, 6A and 6B. The light source 50, 50' may be movable and controlled to point towards the head of the person who is to be awakened.

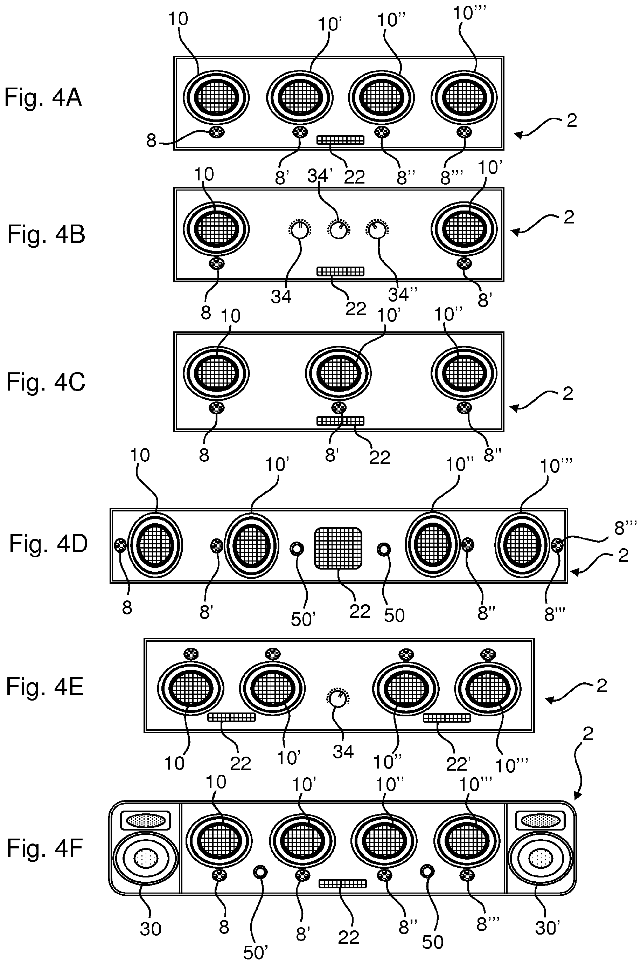

Fig. 4A illustrates a noise reduction system 2 according to the invention. The noise reduction system 2 comprises a box-shaped housing having four directional speaker units 10, 10', 10", 10"' arranged in line (along the length of the housing of the noise reduction system 2). A directional microphone 8, 8', 8", 8"' is arranged below each of the four directional speaker units 10, 10', 10", 10"'. Furthermore, the noise reduction system 2 comprises a centrally arranged sensor 22 configured to detect the position of the head of a person.

Fig. 4B illustrates another noise reduction system 2 according to the invention. The noise reduction system 2 comprises a box-shaped housing corresponding to the housing shown in Fig. 4A. Two directional speaker units 10, 10' are arranged along the length of the housing. A directional microphone 8, 8' is arranged below each of the two directional speaker units 10, 10'. The noise reduction system 2 comprises a centrally arranged sensor 22 adapted to determine the position of the head of a person. Three adjustment members 34, 34', 34" are arranged between the directional speaker units 10, 10'. The adjustment members 34, 34', 34" may be used to change settings (e.g. the position and/or direction to which the directional speaker units 10, 10' and/or the directional microphones 8, 8' point). Accordingly, the noise reduction system 2 may be individually adjusted by means of the adjustment members 34, 34', 34".

Fig. 4C illustrates a further noise reduction system 2 according to the invention. The noise reduction system 2 comprises a box-shaped housing having three directional speaker units 10, 10', 10" arranged in line along the length of the housing of the noise reduction system 2. One directional microphone 8, 8', 8" is arranged below each of the three directional speaker units 10, 10', 10". The noise reduction system 2 is provided with a centrally arranged sensor 22 that is configured to detect the position of the head of a person.

Fig. 4D illustrates an even further noise reduction system 2 according to the invention. The noise reduction system 2 comprises a box-shaped housing provided with four directional speaker units 10, 10', 10", 10"' arranged along the length of the housing of the noise reduction system 2. One directional microphone 8, 8', 8", 8'" is arranged next to each of the four directional speaker units 10, 10', 10", 10"'. The noise reduction system 2 comprises a centrally arranged sensor 22 adapted to detect the position of the head of a person.

Fig. 4E illustrates another noise reduction system 2 according to the invention. The noise reduction system 2 comprises a box-shaped housing equipped with four directional speaker units 10, 10', 10", 10"' arranged along the length of the housing of the noise reduction system 2. One directional microphone 8, 8', 8", 8"' is arranged above each of the four directional speaker units 10, 10', 10", 10"'. The noise reduction system 2 comprises a first sensor 22 arranged below the first and second directional speaker units 10, 10'. The noise reduction system 2 additionally comprises a second sensor 22' arranged below the third and fourth directional speaker units 10", 10"'. The sensors 22, 22' are configured to detect the position of the head of a person. The noise reduction system 2 comprises a centrally arranged adjustment member 34 that is arranged between the second and third directional speaker units 10', 10". The adjustment member 34 can be used to change settings such as the position and/or direction to which the directional speaker units 10, 10', 10", 10"' and/or the directional microphones 8, 8', 8", 8"' are pointing. Therefore, individual adjustments can be made by means of the adjustment member 34.

Fig. 4F illustrates a noise reduction system 2 that basically corresponds to the noise reduction system 2 shown in Fig 4A. The noise reduction system 2, however, comprises two sets of additional hi-fi speakers 30, 30'. The additional hi-fi speakers 30, 30' are arranged in each opposing end regions of the housing of the noise reduction system 2.

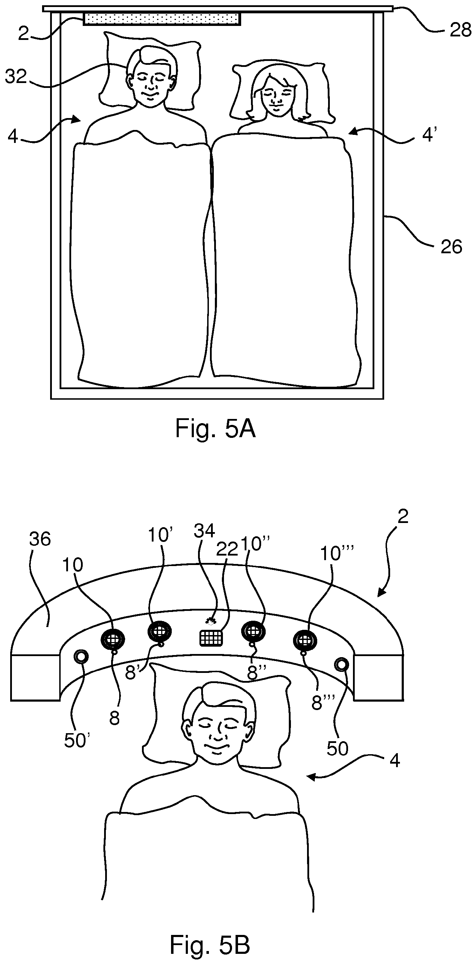

Fig. 5A illustrates a top view of a bed 26 having a headboard 28, wherein a noise reduction system 2 according to the invention is mounted on the headboard 28. A first person 4 is sleeping in the bed 26. Another person 4', is sleeping next to the first person 4. Since the second person 4' is suffering from snoring, the noise reduction system 2 is arranged in the opposite side of the bed 26 (the left-side of the bed 26, where the first person is sleeping).

When the second person 4' starts snoring, the noise reduction system 2 determines the position of the head 32 of the first person 4 by means of a sensor adapted to this purpose. When the position of the head 32 has been detected, the processing unit of the noise reduction system 2 determines which (one or more) of the location specific sound signals, picked up by the directional microphones of the noise reduction system 2, are to be used in order to generate and emit the most effective cancellation signal for providing the most optimum noise reduction. The processing unit of the noise reduction system 2 determines which of the directional speaker units of the noise reduction system 2 that should be used for emitting the most effective cancellation signal. The noise reduction system 2 emits a cancellation signal that reduces the noise originating from the snoring of the second person 4'.

As mentioned, the most optimum noise reduction and most effective cancellation sound may be determined from an energy estimate of the resulting sound after the attempted noise cancellation or it may be determined according to a desired frequency weighting scheme.

Fig. 5B illustrates a noise reduction system 2 according to the invention comprising an arced housing 36. The housing 36 is equipped with four directional speaker units 10, 10', 10", 10"' provided on the arced front surface of the housing 36. One directional microphone 8, 8', 8", 8"' is arranged below each of the four directional speaker units 10, 10', 10", 10"'. The noise reduction system 2 comprises a centrally arranged sensor 22 arranged between the second speaker unit 10' and the third speaker unit 10". The noise reduction system 2 also comprises a centrally arranged adjustment member 34 arranged between the second speaker unit 10' and the third speaker unit 10".

The adjustment member 34 is configured to change the position and/or direction to which the directional speaker units 10, 10', 10", 10'" and/or the directional microphones 8, 8', 8", 8"' are pointing. A person 4 is laying in a bed, wherein the noise reduction system 2 is arranged in a short distance from the person 4.

Fig. 6A illustrates a schematic view of a noise reduction system 2 according to the invention. The noise reduction system 2 comprises a box-shaped housing 36 having four directional speaker units 10, 10', 10", 10'" arranged along the length of the housing of the noise reduction system 2. A directional microphone 8, 8', 8", 8"' is arranged above each of the four directional speaker units 10, 10', 10", 10"'. The noise reduction system 2 comprises a centrally arranged sensor 22 configured to detect the position of the head of a person.

A person 4 is laying in a bed and his head 32 is positioned close to the noise reduction system 2. The noise reduction system 2 applies the sensor 22 to optically detect the position of the head 32. The sensor 22 determines the angle θ and preferably an additional angle e.g. the angle between the vertical axis (extending perpendicular to the X axis) and the direction indicated with a dotted line of the head 32. The sensor 22 is preferably configured to detect the distance D between the sensor 22 and the head 32. Hereby, the position of the head 32 can be uniquely determined.

Fig. 6B illustrates a cross-sectional view of a noise reduction system 2 according to the invention. The noise reduction system 2 comprises a housing 36 and three directional speaker units 10, 10', 10". The directional speaker units 10, 10', 10" are arranged to emit cancellation signals towards various directions. The noise reduction system 2 comprises three directional microphones 8, 8', 8" pointing towards different directions. The noise reduction system 2 comprises a centrally arranged optical sensor 22 configured to detect the position of the head of a person (like illustrated in Fig. 6A).

The noise reduction system 2 comprises a printed circuit board (PCB) 40 provided with a processing unit 38. The processing unit 38 is electrically connected to an antenna 48 configured to communicate wirelessly with one or more external devices (not shown). The processing unit 38 is electrically connected to the directional speakers 10, 10', 10" and the directional microphones 8, 8', 8" by electric cables. Likewise, the processing unit 38 is electrically connected to the sensor 22. The processing unit 38 is furthermore electrically connected to a power module 42 that supplies electric power to the PCB. The power module 42 is electrically connected to the mains by means of a power plug 44 that is connected to a power socket 46.

### List of reference numerals

- 2: Noise reduction system
- 4, 4': Person
- 6: Ear
- 8, 8', 8", 8"': Microphone (preferably directional microphone)
- 10, 10', 10", 10"': Speaker unit (preferably directional speaker unit)
- 12: Sound signal (noise to be reduced)
- 14: Cancellation signal
- 16, 18: Cable
- 20: Processing unit (e.g. computer)
- 22, 22': Sensor
- 24: Noise reduced signal
- 26: Bed
- 28: Headboard
- 30, 30': Speaker unit
- 32: Head
- 34, 34', 34": Adjustment member
- 36: Housing
- 38: Processing unit
- 40: Printed circuit board
- 42: Power module
- 44: Plug
- 46: Socket
- 48: Antenna
- 50, 50': Light source
- A: Amplitude
- T: Time
- α, β, θ: Angle
- X, Y, Z: Axis
- D: Distance
- Z₁, Z₂, Z₃, Z₄: Zone

## Claims

1. A noise reduction system (2) for reducing at least one sound signal (12), wherein the noise reduction system (2) comprises:
- several directional microphones (8, 8', 8", 8"') spaced from each other and adapted to detect at least one location specific sound signal (12) to be reduced;
- a signal processing unit (38) adapted to generate at least one cancellation signal (14);
- a plurality of directional speaker units (10, 10', 10", 10"') adapted to emit at least one cancellation signal (14);
- a head detection sensor (22) configured to detect the position of the
head (32) of a user (4, 4');
**characterised in that** the signal processing unit (38) is configured to determine which one or more of the location specific sound signals picked up by the directional microphones (8, 8', 8", 8"') are to be used in order to generate and emit the cancellation signal (14) for providing a noise reduction, wherein the directional microphones (8, 8', 8", 8"') are arranged to pick up sound from different directions, wherein further the signal processing unit (38) is adapted to determine which of the directional speaker units should be used for emitting the cancellation signal (14) and wherein the directional speaker units (10, 10', 10", 10"') are arranged to emit sound towards different directions.

2. A noise reduction system (2) according to claim 1, **characterised in that** the head detection sensor (22) is an optical sensor configured to detect the position of the head (32).

3. A noise reduction system (2) according to claim 2, **characterised in that** the head detection sensor (22) is an infrared optical sensor.

4. A noise reduction system (2) according to one of the preceding claims, **characterised in that** the directional speaker units (10, 10', 10", 10"') and/or the directional microphones (8, 8', 8", 8"') are moveably arranged.

5. A noise reduction system (2) according to claim 64 **characterised in that** the noise reduction system (2) comprises one or more actuators arranged to change the position or orientation of one or more of the directional speaker units (10, 10', 10", 10"') and/or one or more of the directional microphones (8, 8', 8", 8"').

6. A noise reduction system (2) according to one of the preceding claims, **characterised in that** the noise reduction system (2) comprises one or more additional hi-fi speakers (30, 30').

7. A noise reduction system (2) according to one of the preceding claims, **characterised in that** the noise reduction system (2) comprises a sound unit configured to generate an acoustic sound signal to be emitted by at least one directional speaker unit (10, 10', 10", 10"').

8. A method for reducing at least one location specific sound signal (12) picked up by a plurality of directional microphones (8, 8', 8", 8"'), said method comprises the steps of:
- generating, in a signal processing unit (38), at least one cancellation signal (14);
emitting at least on cancellation signal (14) by means of a plurality of directional speaker units (10, 10', 10", 10"'), wherein the method comprises the further step of detecting the position of the head (32) of a user (4, 4') and determining towards which direction the cancellation signal (14) has to be emitted **characterised in that** the signal processing unit (38) determines which one or more of the location specific sound signals (12) picked up by the directional microphones (8, 8', 8", 8"') are to be used in order to generate and emit the cancellation signal (14) for providing a noise reduction and;
- wherein it is determined which ones of the plurality of directional speaker units are to be used in order to emit a cancellation signal (14),
- wherein the directional microphones (8, 8', 8", 8"') pick up sound from different directions and the directional speaker units (10, 10', 10", 10"') emit sound towards different directions.
